# EUROPEAN PATENT APPLICATION

(11) **EP 2 515 115 A2**
(43) Date of publication of application: **24.10.2012**
(21) Application number: 10837798.7
(22) Date of filing: 29.11.2010
(51) Int. Cl.: G01N 33/574, C07K 16/18

(54) **KIT FOR DIAGNOSING PROSTATE CANCER AND DIAGNOSIS METHOD**

(30) Priority: 17.12.2009 KR 20090126422
(71) Applicant: Cell&Bio Co., Ltd., Seoul 135-270 (KR)
(72) Inventor: YOON, Kang Jun, Seoul 135-270 (KR); SON, YoungSook, Seoul 135-244 (KR); HONG, Hyun Sook, Seoul 143-190 (KR); CHOI, Hyeongwon, Seoul 136-062 (KR)
(74) Representative: McConchie, Connor Fraser
(86) International application number: PCT/KR2010/008480
(87) International publication number: WO 2011/074802

(57) **Abstract**

The present invention relates to a kit and method for diagnosing prostate cancer, which use an antibody to prostate-specific antigen (PSA) to detect PSA in human urine. More specifically, the invention relates to a kit for diagnosing prostate cancer, which comprises an antibody to PSA and uses human urine as a sample, and to a method for diagnosing prostate cancer, which comprises brining a human urine sample into contact with an antibody to PSA in order to detect PSA in the sample.

## Description

### Technical Field

The present invention relates to a kit and method for diagnosing prostate cancer, which use an antibody to prostate-specific antigen.

### Background Art

Prostate cancer is the second leading cancer which causes the death of men in USA, and the number of prostate cancer patients in Korea is also increasing. Prostate cancer patients in Korea accounted for only 1.2% of male cancer patients in 1989, but account for 3% of male cancer patients at present. The mortality in prostate cancer patients in Korea in 1990 was only 0.2%, but was 1.6% in 2000. Unlike other cancers, prostate cancer grows very slowly. Accordingly, if prostate cancer can be diagnosed at an early stage, the possibility of treating it can be increased, suggesting that the early diagnosis of prostate cancer is important.

Prostate specific antigen (PSA) is a serine protease that has a molecular weight of about 33 kDa and is expressed in the prostatic epithelium at a high level. It is secreted into the seminal fluid at a concentration of 1-3 g/L. For normal persons, PSA secreted into the seminal fluid degrades seminal vesicle-specific protein to dissolve coagulated seminal fluid forms (Ulf-Hakan Stenman et al., Cancer Biology, 9: 83-93, 1999). For normal prostates, only a very small amount of produced PSA leaks into blood. However, when the prostate enlarges, a significant amount of PSA leaks out of the prostate. In the case of prostate cancer, a large amount of PSA leaks out, thus increasing the blood level of PSA. Thus, PSA can serve as an important indicator of not only prostate cancer, but also prostate abnormalities such as prostatomegaly. Up to date, cases of early diagnosis of prostate cancer by PSA testing have increased rapidly, and after cancers which have been present, including early prostate cancer, have been significantly removed, the blood level of PSA shows a tendency to decrease rapidly (Korean Journal of Urology, 1999).

For normal persons, the blood level of PSA is 0-4 ng/ml. In persons having a blood PSA level of 1-1.5 ng/ml, the risk of development of prostate cancer is about two times higher than that in persons having a blood PSA level of 1.0 ng/ml or less. In addition, persons having a blood PSA level of 2-3 ng/ml, the risk of development of prostate cancer is about 5 times higher than that in persons having a blood PSA level of 1.0 ng/ml or less. As the prostate enlarges with aging, the blood level of PSA also increases with aging and is about 0-3.5 ng/ml in the 50s age group, about 0-4.5 ng/ml in the 60s age group, and about 0-6.5 ng/ml in the 70s age group.

In the case of current diagnostic kit products, patients having a serum PSA level of 3 ng/ml or more are diagnosed as prostate cancer. However, these kit products have disadvantages in that blood collection is required and diagnosis should be performed in hospitals.

Accordingly, the present inventors have conducted extensive studies on a novel kit and method for diagnosing prostate cancer, which overcome the above-described problems of the existing products. As a result, the present inventors have found that the amount of PSA in the urine of prostate cancer patients is significantly smaller than that in the urine of normal persons, and based on this finding, have developed a kit and method for diagnosing prostate cancer, which use urine in place of blood as a sample, thereby completing the present invention.

### Disclosure

### Technical Problem

It is an object of the present invention to provide a kit for diagnosing prostate cancer, which does not require the collection of blood from a subject.

Another object of the present invention is to provide a method for diagnosing prostate cancer, which does not require the collection of blood from a subject.

### Technical Solution

In order to accomplish the above objects, the present invention provides a kit for diagnosing prostate cancer, which comprises an antibody to PSA and uses human urine as a sample.

The present invention also provides a method for diagnosing prostate cancer, which comprises bringing a human urine sample into contact with an antibody to PSA in order to detect PSA in the sample.

The present inventors have surprisingly found that the amount of PSA in the urine of prostate cancer patients is significantly smaller than at in the urine of normal persons. Although a specific mechanism in which the amount of PSA in the urine of prostate cancer patients decreases compared to that in normal persons has not yet been found, it is thought that, in the case of normal persons, PSA produced in the prostate is normally secreted into the urethra and is detected in the urine, but in the case of persons having prostate abnormalities, the secretion of produced PSA into the urethra is not sufficient.

Thus, the kit and method for diagnosing prostate cancer according to the present invention uses urine in place of blood, unlike the prior art, and enables prostate cancer to be diagnosed when the amount of PSA detected in the human urine of a subject by a PSA-specific antigen is smaller than that in normal persons.

Accordingly, the method for diagnosing prostate cancer according to the present invention comprises the steps of: (a) determining the amount of PSA in a sample obtained from the urine of a subject; (b) comparing the amount determined in the step (a) with a reference amount; and (c) diagnosing whether the subject has prostate cancer, based on the result obtained in the step (b).

The method of the present invention is performed *ex vivo* or *in vitro,* and preferably *in vitro.* In addition, the method may be performed manually or by an automatic device.

As used herein, the term "diagnosing" refers to assessing the development or progression of prostate cancer. As is known to a person skilled in the art, the assessment can be accurately performed for a statistically significant subject, although it is intended to be accurate for 100% of the subjects to be diagnosed. Statistical significance can be easily determined by a person skilled in the art using methods widely known in the art, for example, confidence interval determination, p-value determination, t-test, Mann-Whitney test, etc. Preferred confidence intervals are 90% or higher, 95% or higher, 97% or higher, 98% or higher, and 99%. A preferred p-value is 0.1, 0.05, 0.01, 0.005 or 0.0001. Preferably, diagnosis results according to the present invention will be accurate for 60% or more, 70% or more, 80% or more, or 90% or more of a group of subjects.

As used herein, the term "subject" refers to an animal, preferably a mammal, more preferably a human being.

The sample that is used in the present invention is a urine sample obtained from a subject. The urine sample may be subjected to a pretreatment process such as impurity removal before analysis.

In the present invention, the amount of PSA in the urine sample can be determined by any method known in the art. The method for determining the amount of PSA in the urine sample comprises the steps of: (a) binding PSA in the sample to a PSA-specific antibody, (b) optionally removing the unbound antibody, and (c) determining the amount of the bound PSA-specific antibody. The bound antibody directly or indirectly produces a specific signal by itself or an additional operation.

As used herein, the term "amount" may be not only an absolute amount, but also a relative amount, a concentration, and other parameters derived therefrom.

As used herein, the term "comparing" means comparing the amount of PSA in the urine sample to the amount of PSA in a suitable reference sample. The term "comparing" means the comparison of corresponding parameters. For example, an absolute amount is compared to a suitable reference amount, and a concentration is compared to a reference concentration. Comparing in the step (b) may be carried manually or using a computer. For computer-assisted comparison, the value of the determined amount may be compared to values corresponding to suitable references which are stored in a database by a computer program.

The term "reference amount" as used herein refers to an amount which allows assessing whether a subject has prostate cancer. Accordingly, the reference is obtained from a normal subject who has no prostate cancer. If the amount of PSA in the urine sample of a test subject is similar to or larger than that in the reference sample (i.e., the urine sample of a normal person), the subject is assessed as having no prostate cancer. On the other hand, if the amount of PSA in the urine sample of a test subject is smaller or significantly smaller than that in the reference sample (i.e., the urine sample of a normal person), the test subject is assessed as having prostate cancer. A suitable reference amount can be determined from a reference sample. In a preferred example described below, the amount of PSA in the urine of a normal person was measured to be as large as 50-80 ng/ml, whereas the amount of PSA in a prostate cancer patient was measured to be only 5 ng/ml or less. In addition, the average value of the amounts of PSA in the bloods of normal persons was only 1.0615 pg/ml, whereas the amounts of PSA in prostate cancer patients varied in the range from 20 pg/ml to 800 pg/ml, and the average value thereof reached 332.5 pg/ml. From such results, it can be determined that, in the case of a prostate cancer patient, the level of PSA in the blood significantly increases, whereas the secretion of PSA into the urine shows a tendency to decrease rather than increase, and thus, if the amount of PSA in the urine is significantly smaller than that in the urine of a normal person, the subject can be assessed as having prostate cancer.

Thus, in a specific embodiment of the present invention, a subject can be diagnosed as having prostate cancer when the amount of PSA detected in the urine sample by a PSA-specific antibody is about 10 ng/ml or less. Preferably, a subject can be diagnosed as having prostate cancer when the amount of PSA detected in the urine sample by the PSA-specific antibody is about 7 ng/ml or less.

In another embodiment of the present invention, in the case in which a prostate cancer patient is selected as a reference, if the amount of PSA in a urine sample obtained from a subject is equal or similar to that in a urine sample obtained from the prostate cancer patient, the subject can be diagnosed as having prostate cancer.

The PSA-specific antibody that is used in the present invention is not specifically limited and may be a commercially available anti-PSA antibody or an antibody produced according to a known method. Preferably, the antibody that is used in the present invention may be one or more antibodies selected from the group consisting of HB-10494, HB-9119, HB-11426, HB-8051, HB-8525, HB-8527 (American Type Culture Collection, VA, USA) and anti-PSA Ab (cat no: MO-C40081C: C-PSA1, Abazyme, Needham, MA, USA). In addition, the antibody can be modified to increase the affinity of binding to PSA and may be a monoclonal antibody, a polyclonal antibody, or a fragment thereof which can bind to PSA.

In the present invention, PSA in a urine sample forms an immunological complex through an antigen-antibody reaction with a PSA-specific antibody. The formed immunological complex can be detected by an immunoassay. Numerous methods which are used to detect and/or quantify an antigen are known to those skilled in the art (see Harlow and Lane, Antibodies: A Laboratory Manual, Cold Spring Harbor Laboratory, New York 1988, 556-612]. Specific examples of the immune assay include, but are not limited to, immunochromatography, Western blot, enzyme-linked immunosorbent assay (ELISA), immunohistochemistry, immunocytochemistry and the like. A reagent composition which is used in the immunoassay includes a suitable carrier, a detection label capable of producing a detectable signal, a dissolving agent, and a cleaner. If the detection label is an enzyme, the reagent composition may further include a substrate and a reaction stopper. Suitable carriers include soluble carriers, for example, physiologically acceptable buffers known in the art (e.g., PBS), or insoluble carriers, for example, polystyrene, polyethylene, polypropylene, polyester, polyacrylonitrile, fluorine resin, cross-linked dextran, polysaccharides, polymers such as magnetic microparticles made of latex coated with a metal, paper, glass, metals, agarose, and combinations thereof.

In the present invention, a detection label capable of producing a detectable signal can be conjugated to an antibody to PSA. Examples of the detection label include various enzymes, prosthetic groups, fluorescent materials, luminescent materials, bioluminescent materials, and radioactive materials. The detection label may be coupled or conjugated either directly to the antibody to PSA or indirectly, through an intermediate (such as, for example, a linker known in the art), other polyclonal antibody which can bind to PSA protein, or secondary antibody which can bind to PSA antibody, using techniques known in the art. Examples of suitable enzymes include horseradish peroxidase, acetylcholinesterase, peroxidase, alkaline phosphatase, beta-D-galactosidase, glucose oxidase, maleate dehydrogenase, glucose-6-phosphate dehydrogenase, or invertase or; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, phycoerythrin, or phycobiliprotein; examples of a luminescent material include luminol, isolucinol and lucigenin; examples of bioluminescent materials include luciferase, luciferin, and aequorin; and examples of suitable radioactive materials include ¹²⁵I, ¹³¹I, ¹¹¹In, ⁹⁹Tc ¹⁴C, and ³H. Examples of suitable color developing materials include gold, carbon, and latex.

The inventive kit for diagnosing prostate cancer may comprise the above-mentioned elements for diagnosing prostate cancer in a subject. Specifically, the inventive kit for diagnosing prostate cancer may comprise a container containing an antibody to PSA, a suitable carrier, a detection label capable of producing a detectable signal, a dissolving agent, a cleaner, and the like. When the label is an enzyme, the kit may comprise a substrate callable of measuring enzymatic activity, and a reaction stopper, and the detection label may be coupled or conjugated either directly or through a linker to an antibody to PSA or may be coupled or conjugated to a secondary antibody that recognizes the PSA antibody. Preferably, the kit further includes an instruction for use. Specifically, the kit includes an instruction for use that instructs the user on how to analyze test results in connection with the diagnosis results provided by the method of the present invention. The instruction for use includes information that enables to determine the presence or absence of prostate cancer based on the determined amount of PSA. The user manual may be in the form of a paper or electron file (e.g., CD ROM).

In addition, the inventive kit for diagnosing prostate cancer may use the principle of a currently commercially available kit for diagnosing prostate cancer, designed based on immunochromatographic assay. The immunochromatographic assay is a combination of immunochemistry and chromatography, and it is applied with a specific immune reactivity of an antibody to an antigen, the coloring characteristic and mobility of colloidal gold, and molecule's movement by the capillary phenomenon of a porous membrane. In the immunochromatographic assay, sample dilution required in existing multi-step immune measurement methods, cleaning, and a coloring process which is performed through a reaction between an enzyme complex and a substrate, can be integrated into one system so that a test can be executed fast and conveniently in one step. In addition, test results can be decided without any specific equipment, and thus advantages of easiness, economic feasibility and fast interpretation of test results are provided.

In a preferred embodiment of the present invention, PSA in a human urine sample can be quantified by adding the human urine sample to a microtiter plate coated with an antibody to PSA, reacting the sample with the antibody, reacting the sample with an enzyme-secondary antibody conjugate, treating the sample with a substrate specific for the enzyme to measure the absorbance of the sample, and comparing the absorbance with a standard curve. Biotin can be conjugated to the secondary antibody in the enzyme-secondary antibody conjugate. The enzyme may be horseradish peroxidase, but is not limited thereto. Any enzyme and substrate which are used in ELISA assays may be used in the present invention.

The ELISA assay is an assay method in which an antigen-antibody reaction is used to detect the presence of an antigen in a sample and quantify the antigen. The number of antibodies required in one ELISA kit is 2 or 3. The ELISA kit utilizing antibodies to PSA comprises 2 antibodies, and the construction and principle thereof are as follows.

PSA in a PSA-containing human urine sample can be quantified by coating a 96-well plate with an antibody (primary antibody) to PSA, adding the sample to the well plate, reacting the sample with the antibody, reacting an enzyme-labeled polyclonal antibody (secondary antibody) with the sample, treating the sample with a substrate capable of reacting with the enzyme label, detecting an enzyme-substrate reaction, and comparing the reaction with a standard curve. In a specific embodiment of the present invention, biotin and streptavidin-conjugated horseradish peroxidase may be used as the enzyme and the substrate.

### Advantageous Effects

The kit and method for diagnosing prostate cancer according to the present invention use human urine as a sample, in which the human urine sample is easier to collect than blood which is used in conventional methods. In addition, if the amount of PSA in the urine of a subject is smaller than that in a normal person, the subject can be diagnosed as having prostate cancer. Thus, when the diagnostic kit and method of the present invention are used, the diagnosis of prostate cancer can be easily performed even at home without having to go to a hospital.

### Description of Drawings

FIG. 1 shows the results of quantifying PSA in human urine using an antibody to PSA.
FIG. 2 shows the results of quantifying PSA in human blood using an antibody to PSA.

### Mode for Invention

Hereinafter, the present invention will be described in further detail with reference to examples. It is to be understood, however, that these examples are for illustrative purposes only and are not intended to limit the scope of the present invention.

### Examples

### Example 1: Quantification of PSA in human urine using antibody to PSA

Urine was collected from each of 20 normal persons and 10 prostate cancer patients and centrifuged to remove the precipitate. PSA in the urine samples was quantified using ELISA (Abazyme cat no EL10005) in the following manner.
1) A plate in an ELISA kit was coated with an anti-human PSA monoyclonal antibody (cat no: MO-C40081C: C-PSA1, Abazyme, Needham, MA, USA), and 50 *µℓ* of each of the samples was added to each well.
2) The sample in each well was agitated to spread, and then stored at 37 °C for 30 minutes.
3) Each well was washed 5 times with distilled water.
4) The sample in each well was treated with 100 *µℓ* of a horseradish peroxidase-conjugated secondary antibody (Abazyme, Needham, MA, USA) and incubated at 37 °C for 30 minutes.
5) Each well was washed 5 times with distilled water.
6) The sample in each well was treated with 100 *µℓ* of a substrate solution, and then incubated at 37 °C for 15 minutes.
7) As the antigen-antibody reaction in the sample occurred, a reaction stopper was added to each well to stop the reaction.
8) The degree of color development in each sample was read using an ELISA reader at a wavelength of 450 nm.

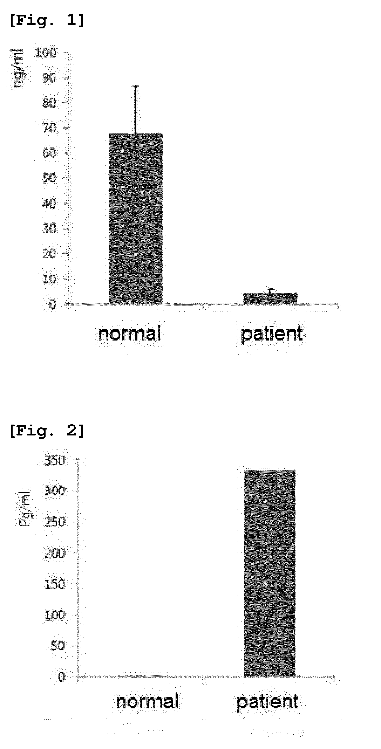

The results of the measurement indicated that the average value of the amounts of PSA in the 20 normal persons was 50-80 ng/ml and the average value of the amounts of PSA in the 10 prostate cancer patients was 5 ng/ml or less (FIG. 1).

### Example 2: Quantification of PSA in human blood using antibody to PSA

3 ml of blood was collected from each of 20 normal persons and 10 prostate cancer patients and centrifuged at 10000 rpm for 5 minutes to precipitate the red blood cell clot, and the supernatant sera were separated. PSA in each serum was quantified using ELISA (Abazyme cat no EL10005) in the same manner as Example 1. The results of the measurement indicated that the average value of the amounts of PSA in the 20 normal persons was only 1.0615 pg/ml, whereas the amounts of PSA in the 10 prostate cancer patients varied in the range from 20 pg/ml to 800 pg/ml, and the average value thereof reached 332.5 pg/ml (FIG. 2).

From the results of Examples 1 and 2, it could be seen that, in the case of the prostate cancer patients, the amount of PSA in the blood significantly increased, whereas the secretion of PSA into the urine showed a tendency to decrease rather than increase. Thus, if the amount of PSA in the urine of a subject is significantly smaller than that in the urine of a normal person, the subject can be diagnosed as having prostate cancer.

## Claims

1. A method for diagnosing prostate cancer, comprising the steps of:
(a) determining the amount of prostate-specific antigen (PSA) in a sample obtained from the urine of a subject;
(b) comparing the amount determined in the step (a) with a reference amount; and
(c) diagnosing whether the subject has prostate cancer, based on the result obtained in the step (b).

2. The method of claim 1, wherein the method is performed *in vitro.*

3. The method of claim 1 or 2, wherein the amount of PSA in the step (a) is determined using a PSA-specific antibody.

4. The method of any one of claims 1 to 3, wherein the reference amount in the step (b) is the amount of PSA determined for a urine sample collected from a normal person.

5. The method of claim 4, wherein the subject is diagnosed as having prostate cancer, if the amount of PSA in the step (a) is smaller than the reference amount of the step (b).

6. The method of any one of claims 1 to 5, wherein the subject is diagnosed as having prostate cancer, if the amount of PSA in step (a) is about 10 ng/ml or less.

7. A prostate cancer-diagnosing kit for use in the method of any one of claims 1 to 6, wherein the kit comprises a PSA-specific antibody and uses urine as a sample.

8. The kit of claim 7, wherein the kit further comprises an instruction for use which includes information that enables to determine the presence or absence of prostate cancer based on the determined amount of PSA in the urine sample.

9. The kit of claim 7, wherein the kit further comprises an instruction for use indicating that prostate cancer is diagnosed when the determined amount of PSA in the urine sample is about 10 ng/ml or less.
